(19) Europäisches Patentamt

European Patent Office

Office européen des brevets

(11) EP 1 189 580 B1

(12) EUROPEAN PATENT SPECIFICATION

(45) Date of publication and mention
of the grant of the patent:
**05.10.2005 Bulletin 2005/40**

(21) Application number: **00940251.2**

(22) Date of filing: **15.05.2000**

(51) Int Cl.⁷: **A61K 7/06**

(86) International application number:
**PCT/EP2000/004434**

(87) International publication number:
**WO 2001/001934 (11.01.2001 Gazette 2001/02)**

(54) **HAIR TREATMENT COMPOSITIONS**

HAARBEHANDLUNGSMITTEL

COMPOSITIONS DE TRAITEMENT CAPILLAIRE

(84) Designated Contracting States:
**AT BE CH CY DE DK ES FI FR GB GR IE IT LI LU MC NL PT SE**

(30) Priority: **30.06.1999 GB 9915319**

(43) Date of publication of application:
**27.03.2002 Bulletin 2002/13**

(73) Proprietors:
• **UNILEVER PLC**
**London EC4P 4BQ (GB)**
Designated Contracting States:
**CY GB IE**
• **UNILEVER N.V.**
**3013 AL Rotterdam (NL)**
Designated Contracting States:
**AT BE CH DE DK ES FI FR GR IT LI LU MC NL PT SE**

(72) Inventors:
• **Fairley, Peter, Unilever Research Port Sunlight Wirral, Merseyside CH63 3JW (GB)**
• **Steer, David C., Unilever Research Port Sunlight Merseyside CN60 6RS (GB)**

(74) Representative: **Tansley, Sally Elizabeth et al Unilever PLC**
**Unilever Intellectual Property Group**
**Colworth House**
**Sharnbrook**
**Bedford, MK44 1LQ (GB)**

(56) References cited:
WO-A-97/09031
US-A- 5 580 550
US-A- 5 534 265
US-A- 5 658 577

**Description**

Field of Invention

The invention relates to hair treatment compositions for imparting improved fullness, body and volume to the hair, as well as superior conditioning, which compositions contain a particular combination of hydrocarbon materials and oily or fatty materials.

Background of Invention and Prior Art

The use in hair treatment compositions of conditioning agents such as silicone polymers, cationic conditioning agents and oily materials in hair treatment compositions is well known and widely documented in the patent literature. However, a problem associated with such materials is that their use at levels necessary for achieving good tactile and/or visual benefits can make the hair feel limp and unmanageable.

Accordingly, hair fullness, body and volume is generally achieved by application of film-forming materials to the hair, usually after shampooing and/or conditioning, in the form of mousses, gels or sprays. However, the adhesive and/or resinous materials used frequently for style retention can damage dry hair properties, especially hair feel and ease of dry combing. Also, many people require a degree of hair fullness, body and volume improvement without the inconvenience and cost of a separate step.

EP 0 498 119 and EP 567 326 disclose the use of high molecular weight resinous per-alk(en)yl hydrocarbon materials for imparting body and stylability to hair.

WO 97/09031 discloses hair shampoo compositions which provide cleaning and styling performance, and which contain latex polymer particles.

The present inventors have now found that hair treatment compositions containing a particular combination of hydrocarbon materials and oily or fatty materials can impart improved fullness, body, volume and stylability to the hair, as well as superior conditioning performance.

Definition of the Invention

The present invention provides a hair treatment composition comprising, in an aqueous medium, (i) emulsified particles of a high molecular weight hydrocarbon material and (ii) a hair conditioning oily or fatty material.

Detailed Description of the Invention

(i) Emulsified particles of high molecular weight hydrocarbon material

By "high molecular weight" is meant that the weight average molecular weight of the emulsified hydrocarbon material is at least 20,000. Suitably it ranges from 20,000 to 1,000,000, preferably 20,000 to 500,000, most preferably 40,000 to 200,000. These materials are especially effective for imparting improved fullness, body and volume to hair.

A preferred class of high molecular weight hydrocarbon materials are per-alk(en)yl hydrocarbon resins. These term "resin" is intended to encompass those materials which are solid or semi-solid at room temperature, as well as those which are liquids with high or moderate viscosities. The term does not cover oils or other low viscosity materials.

EP 567 326 and EP 498 119 describe suitable peralk(en)yl hydrocarbon resins for imparting stylability and enhanced body to hair. Preferred per-alk(en)yl hydrocarbon materials are polymers of butene, isoprene, terpene and styrene, and copolymers of any combination of these monomers, such as butyl rubber (poly isobutylene-co-isoprene), natural rubber (cis-1,4-polyisoprene) and hydrocarbon resins such as mentioned in the Encyclopedia of Chemical technology by Kirk and Othmer (3rd edition vol.8, pp 852-869), for example aliphatic and aromatic resins and terpene resins.

Especially preferred are polyisobutylene materials of the formula:

$$H_3C-[C(CH_3)_2-CH_2-]_m - R$$

wherein m is 1-5000, preferably 2-2500, and R is:

$$-CH(CH_3)_2 \text{ or } -C(CH_3)=CH_2$$

These materials are available from Presperse, Inc. under the PERMETHYL trade name, from Exxon Chemical under the VISTANEX trade name, and from BASF under the OPANOL trade name. Preferred examples include VISTANEX LM-MH and OPANOL B 15.

Suitable methods of making emulsions of particles of high molecular weight hydrocarbon materials such as polyisobutylene resins are described in EP 567 326 and EP 498 119. The process of EP 567 326 is preferred since it is a direct emulsification process with water and a suitable surfactant emulsifier which avoids the need to use a solvent or carrier which is capable of dissolving or dispersing the high molecular weight hydrocarbon material. Such solvents or carriers (e.g. low molecular weight hydrocarbons) can present safety hazards during processing and can destabilise the final formulations into which they are incorporated.

Emulsified high molecular weight hydrocarbon materials for use in hair treatment compositions of the invention generally have an average particle size in the composition of from about 0.01 to about 100 microns, more typically from about 0.1 to about 10 microns, thought this is not particularly critical. Particle size may be measured by means of a laser light scattering technique, using a 2600D Particle Sizer from Malvern Instruments.

Suitable high molecular weight hydrocarbon emulsions for use in the invention are commercially available in a pre-emulsified form. This is particularly preferred since the pre-formed emulsion can be incorporated into the hair treatment composition by simple mixing.

An example of a suitable pre-formed emulsion is the material PIB 96/003 available from Basildon Chemical. This is an aqueous emulsion of the polyisobutylene material OPANOL B 15 (ex BASF) with anionic and nonionic surfactant emulsifier.

The high molecular weight hydrocarbon material may be present in compositions of the invention as a single material or as a mixture of different high molecular weight hydrocarbon materials, e.g. of different molecular weights.

The amount of high molecular weight hydrocarbon material incorporated into the compositions of the invention depends on the level of fullness, body and volume enhancement desired and the specific material used. A preferred amount is from about 0.01 to about 2% by weight of the total composition although these limits are not absolute. The lower limit is determined by the minimum level to achieve the fullness, body and volume enhancing effect and the upper limit by the maximum level to avoid making the hair unacceptably stiff. We have found that an amount of high molecular weight hydrocarbon material of from 0.2 to 0.5% by weight of the total composition is a particularly suitable level.

Hair conditioning oily or fatty material

Hair conditioning oily or fatty materials are preferred conditioning agents in compositions of the invention for adding shine to the hair and also enhancing dry combing and dry hair feel.

Suitable hair conditioning oily or fatty materials have a viscosity at ambient temperature of about 3 $m^2/s$ (3 million cst) or less, preferably about 2 $m^2/s$ (2 million cst) or less, more preferably about 1.5 $m^2/s$ (1.5 million cst) or less.

Suitable hair conditioning oily or fatty materials are selected from hydrocarbon oils, fatty esters and mixtures thereof.

Hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will preferably contain from about 12 to about 19 carbon atoms. Branched chain hydrocarbon oils can and typically may contain higher numbers of carbon atoms. Also suitable are polymeric hydrocarbons of alkenyl monomers, such as $C_2$ -$C_6$ alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length. The number average molecular weight of such materials can vary widely, but is from 200 to 500, preferably by to 400, more preferably from about 300 to about 350.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A further example of a hydrocarbon polymer is polybutene, such as the copolymer of isobutylene and butene. A commercially available material of this type is L-14 polybutene from Amoco Chemical Co. (Chicago, Ill., U.S.A.).

Particularly preferred hydrocarbon oils are the various grades of mineral oils. Mineral oils are clear oily liquids obtained from petroleum oil, from which waxes have been removed, and the more volatile fractions removed by distillation. The fraction distilling between 250°C to 300°C is termed mineral oil, and it consists of a mixture of hydrocarbons ranging from $C_{16}H_{34}$ to $C_{21}H_{44}$. Suitable commercially available materials of this type include Sirius M85 and Sirius M125, all available from Silkolene.

Suitable fatty esters are characterised by having at least 10 carbon atoms, and include esters with hydrocarbyl chains derived from fatty acids or alcohols, e.g., monocarboxylic acid esters, polyhydric alcohol esters, and di- and tricarboxylic acid esters. The hydrocarbyl radicals of the fatty esters hereof can also include or have covalently bonded thereto other compatible functionalities, such as amides and alkoxy moieties, such as ethoxy or ether linkages.

Monocarboxylic acid esters include esters of alcohols and/or acids of the formula R'COOR in which R' and R independently denote alkyl or alkenyl radicals and the sum of carbon atoms in R' and R is at least 10, preferably at least 20.

Specific examples include, for example, alkyl and alkenyl esters of fatty acids having aliphatic chains with from about 10 to about 22 carbon atoms, and alkyl and/or alkenyl fatty alcohol carboxylic acid esters having an alkyl and/or alkenyl alcohol-derived aliphatic chain with about 10 to about 22 carbon atoms, and mixtures thereof.

The monocarboxylic acid ester need not necessarily contain at least one chain with at least 10 carbon atoms, so long as the total number of aliphatic chain carbon atoms is at least 10. Examples include isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Di- and trialkyl and alkenyl esters of carboxylic acids can also be used. These include, for example, esters of $C_4$-$C_8$ dicarboxylic acids such as $C_1$-$C_{22}$ esters (preferably $C_1$-$C_6$) of succinic acid, glutaric acid, adipic acid, hexanoic acid, heptanoic acid, and octanoic acid. Examples include diisopropyl adipate, diisohexyl adipate, and diisopropyl sebacate. Other specific examples include isocetyl stearoyl stearate, and tristearyl citrate.

Polyhydric alcohol esters include alkylene glycol esters, for example ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol monostearate, ethoxylated propylene glycol monostearate, ,polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, polyoxyethylene sorbitan fatty acid esters and mono-, di-and triglycerides.

Particularly preferred fatty esters are mono-, di- and triglycerides, more specifically the mono-, di-, and tri-esters of glycerol and long chain carboxylic acids such as $C_1$-$C_{22}$ carboxylic acids. A variety of these types of materials can be obtained from vegetable and animal fats and oils, such as coconut oil, castor oil, safflower oil, cottonseed oil, corn oil, olive oil, cod liver oil, almond oil, avocado oil, palm oil, sesame oil, lanolin and soybean oil. Synthetic oils include triolein and tristearin glyceryl dilaurate. Specific examples of preferred materials include cocoa butter and palm stearin.

The hair conditioning oily or fatty material may be present in compositions of the invention as a single material or as a blend.

The hair conditioning oily or fatty material may be incorporated as such into the compositions of the invention. Preferably it is then emulsified in situ, for example by high shear mixing of the composition.

Alternatively, the hair conditioning oily or fatty material may be added in a pre-emulsified form. As described above, the high molecular weight hydrocarbon material (i) may also be added in a pre-emulsified form, indeed this is preferable for ease of formulation. In such a case, where both the high molecular weight hydrocarbon material (i) and the hair conditioning oily or fatty material (ii) are added in a pre-emulsified form, then they may be added as two separate emulsions, or, preferably, in the form of a single emulsion. In such a single emulsion, there may be separate particles of emulsified (i) and emulsified (ii) discretely, or alternatively the emulsified particles themselves may contain both (i) and (ii) together.

The hair conditioning oily or fatty material is typically present at a level of from 0.05% to 10%, preferably from 0.2% to 5%, more preferably from about 0.5% to 3%, by total weight of oily or fatty material based on total weight of the composition.

When either or both of the high molecular weight hydrocarbon material (i) and the hair conditioning oily or fatty material (ii) are added in a pre-emulsified form in the manner described above, the exact quantity of emulsion will of course depend on the concentration of the emulsion, and should be selected to give the desired quantity of (i) and/or (ii) in the final composition.

(iii) Hydrocarbon:Oily/Fatty Material Ratios

The high molecular weight hydrocarbon material (i) and hair conditioning oily or fatty material (ii) are incorporated into compositions of the invention in a hydrocarbon:oily/fatty material weight ratio ranging from 1:10 to 1:1, optimally 1:5 to 1:2.

(iv) Product Form

Hair treatment compositions according to the invention may suitably take the form of shampoos, conditioners, sprays, mousses or lotions. Preferred hair treatment composition forms are shampoos and conditioners.

Shampoo Compositions

A particularly preferred hair treatment composition in accordance with the invention is a shampoo composition.

- Cleansing Surfactant

Such a shampoo composition will comprise one or more cleansing surfactants which are cosmetically acceptable and suitable for topical application to the hair. Further surfactants may be present as an additional ingredient if sufficient for cleansing purposes is not provided as emulsifying agent for oily or hydrophobic components (such as silicones) which may typically be present in the shampoo.

It is preferred that shampoo compositions of the invention comprise at least one further surfactant (in addition to that used as emulsifying agent) to provide a cleansing benefit.

Suitable cleansing surfactants, which may be used singularly or in combination, are selected from anionic, amphoteric and zwitterionic surfactants, and mixtures thereof. The cleansing surfactant may be the same surfactant as the emulsifier, or may be different.

Examples of anionic surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di-and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic surfactants for use in shampoos of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, triethanolamine monolauryl phosphate, sodium lauryl ether sulphate 1 EO, 2EO and 3EO, ammonium lauryl sulphate and ammonium lauryl ether sulphate 1EO, 2EO and 3EO.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

The shampoo composition can also include co-surfactants, to help impart aesthetic, physical or cleansing properties to the composition. A preferred example is a nonionic surfactant, which can be included in an amount ranging from 0% to about 5% by weight based on total weight.

For example, representative nonionic surfactants that can be included in shampoo compositions of the invention include condensation products of aliphatic ($C_8$ - $C_{18}$) primary or secondary linear or branched chain alcohols or phenols with alkylene oxides, usually ethylene oxide and generally having from 6 to 30 ethylene oxide groups.

Other representative nonionics include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

$$RO - (G)_n$$

wherein R is a branched or straight chain alkyl group which may be saturated or unsaturated and G is a saccharide group.

R may represent a mean alkyl chain length of from about $C_5$ to about $C_{20}$. Preferably R represents a mean alkyl chain length of from about $C_8$ to about $C_{12}$. Most preferably the value of R lies between about 9.5 and about 10.5. G may be selected from $C_5$ or $C_6$ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix (TRADE MARKS) NS10 ex Seppic; Plantaren (TRADE MARK) 1200 and Plantaren (TRADE MARK) 2000 ex Henkel.

The total amount of surfactant (including any co-surfactant, and/or any emulsifying agent) in shampoo compositions of the invention is generally from 0.1 to 50% by weight, preferably from 5 to 30%, more preferably from 10% to 25% by weight of the total shampoo composition.

- Cationic Polymer

A cationic polymer is a preferred ingredient in shampoo compositions of the invention, for enhancing conditioning performance of the shampoo. Typically such a polymer enhances deposition of conditioning components such as silicone from the shampoo composition onto the intended site during use, i.e. the hair and/or the scalp.

The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:

- copolymers of 1-vinyl-2-pyrrolidine and 1-vinyl-3-methyl-imidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);

- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);

- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;

- mineral acid salts of amino-alkyl esters of homo-and copolymers of unsaturated carboxylic acids having from 3 to 5 carbon atoms, (as described in U.S. Patent 4,009,256);

- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include those of the formula:

$$A\text{-}O\text{-}[R\text{-}N^+(R^1)(R^2)(R^3)X^-]\,,$$

wherein: A is an anhydroglucose residual group, such as a starch or cellulose anhydroglucose residual. R is an alkylene, oxyalkylene, polyoxyalkylene, or hydroxyalkylene group, or combination thereof. $R^1$, $R^2$ and $R^3$ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in $R^1$, $R^2$ and $R^3$) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e. g. as described in U.S. Patent 3,962,418), and copolymers of etherified cellulose and starch (e.g. as described in U. S. Patent 3,958,581).

A particularly suitable type of cationic polysaccharide polymer that can be used is a cationic guar gum derivative, such as guar hydroxypropyltrimonium chloride (Commercially available from Rhone-Poulenc in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic polymer is selected from cationic cellulose and cationic guar derivatives. Particularly preferred cationic polymers are JAGUAR C13S, JAGUAR C15, JAGUAR C17 and JAGUAR C16 and JAGUAR C162.

Conditioners

Compositions in accordance with the invention may also be formulated as conditioners for the treatment of hair (typically after shampooing) and subsequent rinsing.

- Conditioning Surfactant

Such a conditioner will comprise one or more conditioning surfactants which are cosmetically acceptable and suitable for topical application to the hair.

Suitable conditioning surfactants are selected from cationic surfactants, used singly or in admixture. Examples include quaternary ammonium hydroxides or salts thereof, e.g. chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as DEHYQUART (TRADE MARK) ex Henkel.

In conditioners of the invention, the level of cationic surfactant is preferably from 0.01 to 10%, more preferably 0.05 to 5%, most preferably 0.1 to 2% by weight of the composition.

- Fatty Alcohol

Conditioners of the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed.
Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol material in conditioners of the invention is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

(v) Emulsified particles of insoluble silicone

As an optional ingredient in compositions of the invention, particularly shampoos and conditioners, it is preferred to add emulsified particles of insoluble silicone.

The silicone is insoluble in the aqueous matrix of the compositions of the invention and so is present in an emulsified form, with the silicone present as dispersed particles.

Suitable silicones include polydiorganosiloxanes, in particular polydimethylsiloxanes which have the CTFA designation dimethicone.

Also suitable for use in compositions of the invention are hydroxyl functional silicones, in particular polydimethyl siloxanes having hydroxyl end groups which have the CTFA designation dimethiconol.

Also suitable for use in compositions of the invention are silicone gums having a slight degree of cross-linking, as are described for example in WO 96/31188. These materials can impart body, volume and stylability to hair, as well as good wet and dry conditioning.

A further preferred class of silicones for inclusion in shampoos of the invention are amino functional silicones. By "amino functional silicone" is meant a silicone containing at least one primary, secondary or tertiary amine group, or a quaternary ammonium group.

Examples of suitable amino functional silicones include:

(i) polysiloxanes having the CTFA designation "amodimethicone", and the general formula:

$$HO\text{-}[Si(CH_3)_2\text{-}O\text{-}]_x\text{-}[Si(OH)(CH_2CH_2CH_2\text{-}NH\text{-}CH_2CH_2NH_2)\text{-}O\text{-}]_y\text{-}H$$

in which x and y are numbers depending on the molecular weight of the polymer, generally such that the molecular weight is between about 5,000 and 500,000.

(ii) polysiloxanes having the general formula:

$$R'_aG_{3\text{-}a}\text{-}Si(OSiG_2)_n\text{-}(OSiG_bR'_{2\text{-}b})_m\text{-}O\text{-}SiG_{3\text{-}a}\text{-}R'_a$$

in which:

G is selected from H, phenyl, OH or $C_{1\text{-}8}$ alkyl, e.g. methyl;
a is 0 or an integer from 1 to 3, preferably 0;
b is 0 or 1, preferably 1;
m and n are numbers such that (m + n) can range from 1 to 2000, preferably from 50 to 150;
m is a number from 1 to 2000, preferably from 1 to 10;
n is a number from 0 to 1999, preferably from 49 to 149, and

R' is a monovalent radical of formula $-C_qH_{2q}L$ in which q is a number from 2 to 8 and L is an aminofunctional group selected from the following:

$$-NR''\text{-}CH_2\text{-}CH_2\text{-}N(R'')_2$$

$$-N(R'')_2$$

$$-N^+(R'')_3A^-$$

$$-N^+H(R'')_2 \ A^-$$

$$-N^+H_2(R'') \ A^-$$

$$-N(R'')-CH_2-CH_2-N^+H_2(R'') \ A^-$$

in which R'' is selected from H, phenyl, benzyl, or a saturated monovalent hydrocarbon radical, e.g. $C_{1-20}$ alkyl, and A is a halide ion, e.g. chloride or bromide.

Suitable amino functional silicones corresponding to the above formula include those polysiloxanes termed "trimethylsilylamodimethicone" as depicted below, and which are sufficiently water insoluble so as to be useful in compositions of the invention:

$$Si(CH_3)_3 - O - [Si(CH_3)_2 - O - ]_x - [Si \ (CH_3) \ (R - NH -$$

$$CH_2CH_2 \ NH_2) - O -]_y - Si \ (CH_3)_3$$

wherein x + y is a number from about 50 to about 500, and wherein R is an alkylene group having from 2 to 5 carbon atoms. Preferably, the number x + y is in the range of from about 100 to about 300.

(iii) quaternary silicone polymers having the general formula:

$$\{(R^1) \ (R^2) \ (R^3) \ N^+ \ CH_2CH(OH)CH_2O(CH_2)_3[Si(R^4) \ (R^5)-O-]_n-Si(R^6) \ (R^7)-$$

$$(CH_2)_3-O-CH_2CH(OH)CH_2N^+ \ (R^8) \ (R^9) \ (R^{10})\} \ (X^-)_2$$

wherein $R^1$ and $R^{10}$ may be the same or different and may be independently selected from H, saturated or unsaturated long or short chain alk(en)yl, branched chain alk(en)yl and $C_5$-$C_8$ cyclic ring systems;

$R^2$ thru' $R^9$ may be the same or different and may be independently selected from H, straight or branched chain lower alk(en)yl, and $C_5$-$C_8$ cyclic ring systems;
n is a number within the range of about 60 to about 120, preferably about 80, and
$X^-$ is preferably acetate, but may instead be for example halide, organic carboxylate, organic sulphonate or the like.
Suitable quaternary silicone polymers of this class are described in EP-A-0 530 974.
Amino functional silicones suitable for use in shampoos of the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.
Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).
An example of a quaternary silicone polymer useful in the present invention is the material K3474, ex Goldschmidt.
For dimethicone and dimethiconol-type silicones, the viscosity of the silicone itself (not the emulsion of which it forms part, in the case of a pre-emulsified silicone, or the final hair treatment composition) is typically at least 10,000 cst, preferably at least 60,000 cst, most preferably at least 500,000 cst, ideally at least 1,000,000 cst. Preferably the viscosity does not exceed $10^9$ cst for ease of formulation. For amino functional-type silicones, the viscosity of the silicone itself is not particularly critical and can suitably range from about 100 to about 500,000 cst.
Emulsified silicones for use in hair treatment compositions of the invention will typically have an average silicone particle size in the composition of less than 30, preferably less than 20, more preferably less than 10 microns. In general, reducing the silicone particle size tends to improve conditioning performance. Most preferably the average silicone particle size of the emulsified silicone in the composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Suitable silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

Pre-formed emulsions of amino functional silicone are also available from suppliers of silicone oils such as Dow Corning and General Electric. Particularly suitable are emulsions of amino functional silicone oils with non ionic and/or cationic surfactant. Specific examples include DC929 Cationic Emulsion, DC939 Cationic Emulsion, DC949 Cationic emulsion, and the non-ionic emulsions DC2-7224, DC2-8467, DC2-8177 and DC2-8154 (all ex Dow Corning).

Mixtures of any of the above types of silicone may also be used. Particularly preferred are hydroxyl functional silicones, amino functional silicones and mixtures thereof.

The total amount of silicone incorporated into compositions of the invention depends on the level of conditioning desired and the material used. A preferred amount is from 0.01 to about 10% by total weight of silicone based on the total weight of the composition although these limits are not absolute. The lower limit is determined by the minimum level to achieve conditioning and the upper limit by the maximum level to avoid making the hair and/or skin unacceptably greasy.

When the silicone is incorporated as a pre-formed emulsion as described above, the exact quantity of emulsion will of course depend on the concentration of the emulsion, and should be selected to give the desired quantity of silicone in the final composition.

(vi) Further optional ingredients

Compositions of this invention may contain any other ingredient normally used in hair treatment formulations. These other ingredients may include, viscosity modifiers, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

The invention is further illustrated by way of the following non-limitative Examples, in which all percentages are by weight based on total weight unless otherwise stated.

EXAMPLES

Shampoo formulations were made up having ingredients as shown in the following Table. Examples 2, 4, 5 and 6 are Examples according to the invention whereas Examples 1 and 3 are Comparative Examples.

| EXAMPLE | 1 | 2 | 3 | 4 | 5 | 6 |
|---|---|---|---|---|---|---|
| INGREDIENT | {% active ingredient] | | | | | |
| Sodium laureth sulphate 2EO | 14.0 | 14.0 | 140 | 14.0 | 11.0 | 11.0 |
| Cocoamidopropyl betaine | 2.0 | 2.0 | 2.0 | 2.0 | 3.0 | 3.0 |
| Timiron MP1001 | 0.2 | 0.2 | 0.2 | 0.2 | - | - |
| Agar | 0.6 | 0.6 | - | - | - | - |
| Carbopol 980 | - | - | 0.4 | 0.4 | 0.4 | 0.4 |
| Jaguar C13S | 0.1 | 0.1 | 0.1 | 0.1 | 0.1 | 0.1 |
| Polyisobutylene (VISTANEX LM-MH ex Exxon) | 0.25 | 0.25 | 0.25 | 0.25 | 0.4 | 0.5 |
| Sirius M85 mineral oil (ex Silkolene) | - | 1.0 | - | - | - | - |
| Sirius M125 mineral oil (ex Silkolene) | - | - | - | 1.5 | 0.8 | 1.0 |

(continued)

| EXAMPLE | 1 | 2 | 3 | 4 | 5 | 6 |
|---|---|---|---|---|---|---|
| INGREDIENT | {% active ingredient] | | | | | |
| Peanut oil | - | - | - | - | 1.0 | - |
| Castor oil | - | - | - | - | - | 0.5 |
| Sodium benzoate | 0.5 | 0.5 | 0.5 | 0.5 | 0.5 | - |
| Phenoxyethanol | 0.4 | 0.4 | 0.4 | 0.4 | 0.4 | 0.4 |
| PPG400 | 0.2 | - | - | - | - | - |
| Fragance | 0.55 | 0.55 | 0.55 | 0.55 | 0.55 | 0.55 |
| Sodium chloride | 1.3 | 1.3 | 1.0 | 1.0 | 1.0 | 1.0 |
| Water | ----------------to 100.0----------------- | | | | | |

In Examples 2,4, 5 and 6 the polyisobutylene was dissolved in the mineral oil and emulsified by high shear mixing prior to incorporation into the shampoo.

A comparative evaluation comparing the formulations of Examples 1 vs. 2 and 3 vs. 4 respectively (i.e. +/- Sirius oil in each case) was obtained from testing by a panel of 100 women with fine hair.

No difference was obtained on styling attributes such as volume, style retention and in achieving the desired style, indicating that the presence of Sirius oil has no deleterious effect on the styling attributes of the polyisobutylene.

On conditioning attributes the following results were obtained:

Examples 2 vs. 1: a significant win for Example 2 over Example 1 on softness (99% sig.) and smoothness (90% sig.)

Examples 4 vs. 3: a significant win for Example 4 over Example 3 on softness (95% sig.) and wet combing (90% sig.). There was also a significant "win" (95% sig.) for Example 3 over Example 4 on the negative attribute of sticky feel after rinsing.

The results indicate that the Sirius oil is delivering conditioning benefits on top of the volume and style retention benefits imparted by the polyisobutylene.

## Claims

1. A hair treatment composition comprising, in an aqueous medium, (i) emulsified particles of a high molecular weight hydrocarbon material and (ii) a hair conditioning oily or fatty material selected from hydrocarbon oils with a number average molecular weight of from 200 to 500, fatty esters having a viscosity at ambient temperature of $3m^2/s$ or less, and mixtures thereof, wherein the weight ratio of (i) to (ii) is in the range from 1:10 to 1:1.

2. A composition according to claim 1, in which the fatty ester is selected from mono-, di-, and tri-esters of glycerol.

3. A composition according to claim 1 or claim 2 in which the hydrocarbon material (i) is a per-alk(en)yl hydrocarbon resin.

4. A composition according to claim 3, in which the peralk(en)yl hydrocarbon resin is a polyisobutylene material of the formula:

$$H_3C \text{ -}[C(CH_3)_2\text{-}CH_2\text{-}]_m \text{ - R}$$

wherein m is 1-5000, preferably 2-2500, and R is:

$$\text{-}CH(CH_3)_2 \text{ or -}C(CH_3)\text{=}CH_2$$

5. A composition according to any one of the preceding claims, which is a hair shampoo composition comprising one or more cleansing surfactants.

6. A composition according to claim 5, further comprising from about 0.01 to about 5% by weight of a deposition aid which is a cationic polymer selected from the group comprising cationic polysaccharide polymers, cationic starch derivatives, cationic guar derivatives and cationic polyacrylamides.

**Patentansprüche**

1. Haarbehandlungszusammensetzung, umfassend in einem wässrigen Medium (i) emulgierte Teilchen eines Kohlenwasserstoffmaterials mit hohem Molekulargewicht und (ii) ein haarkonditionierendes, öliges oder fettiges Material, ausgewählt aus Kohlenwasserstoffölen mit einem zahlenmittleren Molekulargewicht von 200 bis 500, Fettestern mit einer Viskosität bei Umgebungstemperatur von 3 $m^2$/s oder weniger, und Gemischen davon, wobei das Gewichtsverhältnis von (i) zu (ii) im Bereich von 1:10 bis 1:1 liegt.

2. Zusammensetzung nach Anspruch 1, worin der Fettester aus Mono-, Di- und Triestern von Glycerin ausgewählt ist.

3. Zusammensetzung nach Anspruch 1 oder Anspruch 2, worin das Kohlenwasserstoffmaterial (i) ein Peralk(en)ylkohlenwasserstoffharz ist.

4. Zusammensetzung nach Anspruch 3, worin das Peralk(en)ylkohlenwasserstoffharz ein Polyisobutylenmaterial der Formel

$$H_3C\ \text{-[C}(CH_3)_2\text{-}CH_2\text{-]}_m \text{ - R,}$$

worin m 1-5000, vorzugsweise 2-2500, ist und R
-CH(CH_3)_2 oder -C(CH_3)=CH_2 darstellt, ist.

5. Zusammensetzung nach einem der vorangehenden Ansprüche, die eine ein oder mehrere reinigende Tenside umfassende Haarshampoozusammensetzung darstellt.

6. Zusammensetzung nach Anspruch 5, weiterhin umfassend etwa 0,01 bis etwa 5 Gewichtsprozent einer Abscheidungshilfe, die ein kationisches Polymer darstellt, ausgewählt aus der Gruppe, umfassend kationische Polysaccharidpolymere, kationische Stärkederivate, kationische Guarderivate und kationische Polyacrylamide.

**Revendications**

1. Composition pour le traitement des cheveux comprenant, dans un milieu aqueux, (i) des particules émulsionnées d'un matériau hydrocarboné de masse moléculaire élevée et (ii) un matériau huileux ou gras de conditionnement des cheveux choisi parmi les huiles hydrocarbonées ayant une masse moléculaire moyenne en nombre de 200 à 500, les esters gras ayant une viscosité à la température ambiante de 3 $m^2$/s ou moins, et leurs mélanges, dans laquelle le rapport en poids de (i) à (ii) est situé dans la plage allant de 1/10 à 1/1.

2. Composition selon la revendication 1, dans laquelle l'ester gras est choisi parmi les mono-, di- et tri-esters de glycérol.

3. Composition selon la revendication 1 ou la revendication 2, dans laquelle le matériau hydrocarboné (i) est une résine hydrocarbonée peralkylique ou peralcénylique.

4. Composition selon la revendication 3, dans laquelle la résine hydrocarbonée peralkylique ou peralcénylique est un matériau de type polyisobutylène de formule :

$$H_3C\text{- [C}(CH_3)_2\text{-}CH_2\text{-]}_m\text{-R}$$

dans laquelle m vaut 1-5000, de préférence 2-2500, et R est :

$$-CH(CH_3)_2 \text{ ou } -C(CH_3)=CH_2$$

**5.** Composition selon l'une quelconque des revendications précédentes, qui est une composition de shampooing pour les cheveux comprenant un ou plusieurs tensioactifs nettoyants.

**6.** Composition selon la revendication 5, comprenant en outre d'environ 0,01 à environ 5 % en poids d'un auxiliaire de déposition qui est un polymère cationique choisi dans le groupe comprenant les polymères polysaccharidiques cationiques, les dérivés d'amidon cationiques, les dérivés de guar cationiques et les polyacrylamides cationiques.